# EUROPÄISCHE PATENTANMELDUNG

(11) **EP 0 799 653 A2**
(43) Veröffentlichungstag der Anmeldung: **08.10.1997**
(21) Anmeldenummer: 97105072.9
(22) Anmeldetag: 26.03.1997
(51) Int. Cl.: B09C 1/10, C12N 1/20

(54) **Nährstoffsubstrat**

(30) Priorität: 04.04.1996 DE 19613794
(71) Anmelder: Agra Dünger GmbH, 66333 Völklingen (DE)
(72) Erfinder: Litzenburger, Klaus, 66440 Niederwürzbach (DE)

(57) **Zusammenfassung**

Die Erfindung betrifft ein Stoffgemisch zur Verwendung als Nährstoffsubstrat für Mikroorganismen bei der biologischen Bodensanierung, insbesondere dem mikrobiologischen Abbau von Kohlenwasserstoffen.
Das Stoffgemisch besteht aus einem Polymergemisch unterschiedlicher Methylen-Harnstoff-Kondensate mit vorwiegend einfachem Monomercharakter entsprechend Methylen-Diharnstoff und Dimethylen-Triharnstoff und Kondensaten mit komplexerem Monomercharakter entsprechend Trimethylen-Tetraharnstoff und Tetramethylen-Pentaharnstoff.
Besonders günstig wird das erfindungsgemäße Stoffgemisch als Suspension ausgebildet und in die Böden eingebracht.

## Beschreibung

Die Erfindung betrifft ein Stoffgemisch zur Verwendung als Nährstoffsubstrat für Mikroorganismen bei der biologischen Bodensanierung, insbesondere dem mikrobiologischen Abbau von Kohlenwasserstoffen.

Es ist bekannt, daß bei biologischen Verfahren zur Dekontamination von durch organische Verbindungen schadstoffbelasteten Böden die Dekontaminationsrate auch dadurch gesteigert werden kann, daß mineralische Nährsalze in die entsprechenden Böden eingebracht werden. Angestrebt wird dabei eine Vergrößerung der angestammten oder angesiedelten Population von Mikroorganismen, die, ein günstiges Millieu vorausgesetzt, in biotischer Weise die eigentliche Dekontamination bewirken. Die biologische Aktivität derart mit Nährsalzen angereicherter Böden kann dabei um ein mehrfaches gesteigert werden, was sich durch Nachweis der Lebendkeimzahl sowie der Atmungsaktivität quantitativ belegen läßt. Von derart verwendeten NPK-Substraten ist jedoch auch bekannt, daß sich bei einer Überdosierung des Stickstoffzusatzes in Form von z. B. Nitraten oder Ammoniumsalzen oder Harnstoff die Dekontaminationsrate wieder verringert. Es tritt offensichtlich eine biotische Hemmung auf, die auf möglicherweise schon toxische Mengen an Stickstoffverbindungen für die Population der Mikroorganismen zurückzuführen ist. Dem eigentlichen Ziel, einer gesteigerten Dekontaminationsrate, wird damit nicht mehr gedient. Die Bioverfügbarkeit der überdosiert eingebrachten Nährsalze ist so massiv, daß von einer Kontraproduktivität gesprochen werden kann.

Die Problematik der Stickstoffversorgung von Böden und die damit einhergehende Nitratbelastung von Boden und Grundwasser soll ökologisch nicht unerwähnt bleiben.

Aufgabe der Erfindung ist es daher, ein Nährstoffsubstrat zu entwickeln, das nach dem Einbringen in den belasteten Boden eine gleichmäßige und langandauernde Bioverfügbarkeit - im Sinne einer Nährwirkung - auf die Population der Mikroorganismen ausübt und dadurch eine gesteigerte Dekontaminationsrate gewährleistet.

Die Aufgabe wird dadurch gelöst, daß das als Nährstoffsubstrat verwendete Stoffgemisch aus einem Polymergemisch unterschiedlicher Methylen-Harnstoff-Kondensate mit vorwiegend einfachem Monomercharakter entsprechend Methylen-Diharnstoff und Dimethylen-Triharnstoff und Kondensaten mit komplexerem Monomercharakter entsprechend Trimethylen-Tetraharnstoff und Tetramethylen-Pentaharnstoff besteht.

Das erfindungsgemäß angewandte Stoffgemisch schließt aus, daß größere Dosierungen an Nährstoffsubstrat die biotische Aktivität der Mikroorganismenpopulation hemmen. Es gewährleistet eine gesteigerte Dekontaminationsrate und stellt somit sicher, daß der zeitliche Bedarf zur Dekontamination eines vorgegebenen belasteten Bodenareals erheblich verringert wird. Derart gesteigerte Durchsatzraten tragen erheblich zur Wirtschaftlichkeit solcher Anlagen bei, ohne daß die Gefahr erhöhter Nitratbelastungen gegeben ist.

Die erfindungsgemäße Verwendung des Stoffgemisches kombiniert damit eine gesteigerte Wirtschaftlichkeit mit vorbeugendem Umweltschutz. Für den biotischen Wirkmechanismus im belasteten Boden ist es unerheblich in welcher Form, ob als Pulver, Granulat oder Suspension, das Nährstoffsubstrat in das Bodenareal eingebracht wird. Mit dem Einbringen als Suspension wird jedoch in vorteilhafter Weise eine sehr gleichmäßige Substratverteilung im Boden bewerkstelligt, was mittels Pulver und Granulat nur durch aufwendige Mischvorgänge oder Umschüttungen erreicht wird.

## Patentansprüche

1. Stoffgemisch zur Verwendung als Nährstoffsubstrat für Mikroorganismen bei der biologischen Bodensanierung, insbesondere dem mikrobiologischen Abbau von Kohlenwasserstoffen,
**bestehend aus**
einem Polymergemisch unterschiedlicher Methylen-Harnstoff-Kondensate mit vorwiegend einfachem Monomercharakter entsprechend Methylen-Diharnstoff und Dimethylen-Triharnstoff und Kondensaten mit komplexerem Monomercharakter entsprechend Trimethylen-Tetraharnstoff und Tetramethylen-Pentaharnstoff.

2. Stoffgemisch nach Anspruch 1,
**dadurch gekennzeichnet, daß**
das Stoffgemisch als Suspension ausgebildet wird.
